# EUROPÄISCHE PATENTSCHRIFT

(11) **EP 0 821 234 B1**
(45) Veröffentlichungstag und Bekanntmachung des Hinweises auf die Patenterteilung: **09.10.2002**
(21) Anmeldenummer: 97112361.7
(22) Anmeldetag: 18.07.1997
(51) Int. Cl.: G01N 33/52

(54) **Diagnostischer Testträger mit mehrschichtigem Testfeld und Verfahren zur Bestimmung von Analyten mit dessen Hilfe**
Diagnostic test element with multilayered test field and metod to assay analytes using it
Moyen diagnostique avec champ d'épreuve multicouche et méthode de détermination d'analytes l'utilisant

(30) Priorität: 23.07.1996 DE 19629656
(43) Veröffentlichungstag der Anmeldung: 28.01.1998
(73) Patentinhaber: Roche Diagnostics GmbH, 68298 Mannheim (DE)
(72) Erfinder: Thym, Detlef, Dr., 68259 Mammheim (DE); Knappe, Wolfgang-Reinhold, Dr., 67061 Ludwigshafen (DE); Pachl, Rudolf, Dr., 67158 Ellerstadt (DE); Merdes, Hartmut, Dr., 69123 Heidelberg (DE); Lorenz, Robert, 67547 Worms (DE)

(56) Entgegenhaltungen:
- EP-A- 0 302 287
- WO-A-92/15879

## Beschreibung

Die Erfindung betrifft einen diagnostischen Testträger zur Bestimmung eines Analyten aus Vollblut mit Hilfe eines in dem Testträger enthaltenen Reagenzsystems, welches ein Farbbildungsreagenz einschließt, mit einem Testfeld, welches eine Probenaufgabeseite, auf die die Blutprobe zugeführt wird, und eine Nachweisseite, auf der in Folge der Reaktion des Analyten mit dem Reagenzsystem eine optisch nachweisbare Veränderung stattfindet, aufweist und welches so ausgebildet ist, daß die in der Probe enthaltenen Erythrozyten nicht auf die Nachweisseite gelangen. Die Erfindung betrifft außerdem ein Verfahren zur Bestimmung eines Analyten aus Vollblut mit Hilfe eines erfindungsgemäßen diagnostischen Testträgers.

Zur qualitativen oder quantitativen analytischen Bestimmung von Bestandteilen von Körperflüssigkeiten, insbesondere von Blut, werden oft sogenannte trägergebundene Tests verwendet. Bei diesen liegen Reagenzien auf oder in entsprechenden Schichten eines festen Testträgers vor, der mit der Probe in Kontakt gebracht wird. Die Reaktion von flüssiger Probe und Reagenzien führt zu einem nachweisbaren Signal, insbesondere einer Farbänderung, welche visuell oder mit Hilfe eines Gerätes, meistens reflektionsphotometrisch, ausgewertet werden kann.

Testträger sind häufig als Teststreifen ausgebildet, die im wesentlichen aus einer länglichen Tragschicht aus Kunststoffmaterial und darauf aufgebrachten Testfeldern mit einer oder mehreren Nachweisschichten bestehen. Es sind jedoch auch Testträger bekannt, die als quadratische oder rechteckige Blättchen gestaltet sind.

Trägergebundene Tests zeichnen sich insbesondere durch ihre einfache Handhabung aus. Um so bedauerlicher ist es, daß bei vielen bisher bekannten Testträgern Blut als Probenflüssigkeit nicht unmittelbar als Vollblut verwendet werden kann. Es ist vielmehr erforderlich, die roten Blutkörperchen (Erythrozyten) abzutrennen um farbloses Plasma bzw. Serum zu gewinnen. Dies geschieht üblicherweise durch Zentrifugieren. Damit ist jedoch ein zusätzlicher Handhabungsschritt verbunden, der eine verhältnismäßig große Blutmenge und aufwendige Apparatur erfordert.

Es hat daher nicht an Versuchen gefehlt, Testträger zur Verfügung zu stellen, welche analytische Bestimmungen unmittelbar aus Blut ermöglichen. Dabei kann man zwei grundsätzlich verschiedene Lösungmöglichkeiten unterscheiden.

Bei dem ersten Lösungsansatz erfolgt die visuelle oder apparative Auswertung des Farbumschlags auf der gleichen Seite des Testfeldes, auf die auch die Probe aufgegeben wird. Dabei ist das Testfeld so aufgebaut, daß der Analyt aus der Probe durch die Testfeldoberfläche zu den Reagenzien gelangt, während die Erythrozyten zurückgehalten werden. Nach einer definierten Zeit wird die Blutprobe von der Testfeldoberfläche abgewischt oder abgewaschen und der Farbumschlag beobachtet. Beispiele solcher Testträger sind in der US-A-3,630,957 und in der EP-A-0 217 246 beschrieben.

Bei dem zweiten Lösungsansatz wird die Probe auf der einen Seite des Testfeldes aufgegeben (Probenaufgabeseite) und der Farbumschlag auf der anderen Seite (Nachweisseite) registriert. Ein wesentlicher Vorteil dieser Verfahrensweise besteht darin, daß das Blut nicht abgewischt oder abgewaschen werden muß. Man bezeichnet solche Testträger deswegen auch als nicht abzuwischende Testträger (non-wipe test carriers).

Mit dem Abwischen entfällt nicht nur ein lästiger Handhabungsschritt, sondern auch eine mögliche Fehlerquelle, die daraus resultieren kann, daß der Zeitpunkt, zu dem Blut entfernt werden muß, nicht genau eingehalten wird. Andererseits ist dieser Lösungsansatz besonders schwer zu realisieren. Erforderlich ist ein Erythrozytenfilter, der einerseits die intensiv färbenden Bestandteile des Blutes zuverlässig zurückhält, andererseits den Analyt vollständig und ausreichend schnell passieren läßt. Es erweist sich als außerordentlich schwierig, einen Testfeldaufbau zu finden, der diese Anforderungen erfüllt.

Aus der EP-A-0 045 476 ist es bekannt, Glasfasern zur Gewinnung von Serum oder Plasma auf einem Testträger einzusetzen. Diese Lösung ist zwar universell einsetzbar, jedoch ist es erforderlich, die Glasfaserschichten in geeigneter Weise auf dem Testträger unterzubringen. Dadurch entsteht ein verhältnismäßig komplizierter Testträgeraufbau, und das Verfahren der Herstellung ist aufwendig. Außerdem absorbiert die Glasfaserschicht Flüssigkeit, die dann der Nachweisschicht nicht zur Verfügung steht. Dies bedingt einen relativ hohen Probenvolumenbedarf.

In der EP-A-0 302 287 ist ein Testfeld dargestellt, welches einen Schichtverbund aufweist, der durch Flüssigbeschichten einer das Farbbildungsreagenz enthaltenden Nachweisschicht auf eine der Probenaufgabenseite zugewandte Basischicht hergestellt ist. Die Basisschicht enthält einen polymeren Filmbildner, Kieselgur und ein Pigment. Die Nachweisschicht enthält einen polymeren Filmbildner, welcher beim Flüssigbeschichten teilweise in die Basisschicht eindringt und eine Übergangszone bildet, in der die Erythrozyten zurückgehalten werden. Wie sich aus den Beispielen ergibt, ist die pigmenthaltige Schicht jedoch um bis zu 10 mal dicker als die Nachweisschicht. Dies hat erhebliche Auswirkungen auf den Volumenbedarf eines solchen Testfeldes. Da nur die Übergangszone zwischen der pigmenthaltigen und der Nachweisschicht als Erythrozytenrückhaltezone dient, muß ein relativ großes Volumen (der pigmenthaltigen Schicht) mit Flüssigkeit gefüllt werden, bevor die Nachweisschicht mit Flüssigkeit gefüllt wird.

Aus WO 92/15879 ist ein Testträger mit einem einschichtigen, pigmenthaltigen Nachweiselement bekannt, welches gleichzeitig die Funktion einer Blutfarbstoffabtrennschicht erfüllt. Dieses Nachweiselement ist aus einer Dispersion oder Emulsion eines polymeren Filmbildners gebildet, welche in homogener Verteilung das Pigment, den Filmbildner, das Farbbildungsreagenz des Reagenzsystems und ein Quellmittel enthält.

Da keine der vorbekannten Realisierungen eines NW-Testträgers mit Erythrozytenabtrennung in jeder Hinsicht befriedigende Eigenschaften aufweist, wurde bei einer im Handel befindlichen Realisierung eines solchen Testträgers auf die Abtrennung der Erythrozyten vollständig verzichtet und die Störung durch die intensive Rotfärbung meßtechnisch mit Hilfe einer Messung bei zwei verschiedenen Wellenlängen kompensiert. Dabei wird aber der Aufwand für die apparative Auswertung stark erhöht, und es ist keine visuelle Kontrolle des Farbumschlags möglich.

Der Erfindung liegt die Aufgabe zugrunde, einen einfach herzustellenden Testträger zur Verfügung zu stellen, mit dem eine einfache und schnelle Bestimmung von Analyt aus Vollblut durchzuführen ist. Diese Bestimmung soll mit einem möglichst geringen Blutvolumen auskommen, aber dennoch hämatokrit-unabhängig sein. Eine einfache Bestimmung soll eine visuelle Beurteilungsmöglichkeit der Analytkonzentration einschließen.

Diese Aufgabe wird durch die in den Patentansprüchen näher charakterisierte Erfindung gelöst.

Gegenstand der Erfindung ist ein diagnostischer Testträger zur Bestimmung eines Analyten aus Vollblut mit Hilfe eines in dem Testträger enthaltenen Reagenzsystems, welches ein Farbbildungsreagenz einschließt. Der Testtrager enthält ein Testfeld, welches eine Probenaufgabeseite aufweist, der die Blutprobe zugeführt wird, und außerdem eine Nachweisseite besitzt, auf der in Folge der Reaktion des Analyten mit dem Reagenzsystem eine optisch nachweisbare Veränderung stattfindet. Die in Blut enthaltenen Erythrozyten gelangen nicht auf die Nachweisseite. Erfindungsgemäß umfaßt das Testfeld eine transparente Folie, auf die in dieser Reihenfolge eine erste und eine zweite Filmschicht übereinanderliegend aufgebracht sind. Wesentlich ist, daß die auf der transparenten Folie befindliche erste Schicht bedeutend weniger lichtstreuend ist als die darüberliegende zweite Schicht. Die nicht beschichtete Seite der transparenten Folie wird als Nachweisseite bezeichnet und die Seite der zweiten Schicht, die der Seite gegenüberliegt, mit der die zweite Schicht auf der ersten aufliegt, wird als Probenaufgabeseite bezeichnet.

Außerdem ist Gegenstand der Erfindung ein Verfahren zur Bestimmung eines Analyten aus Vollblut mit Hilfe eines erfindungsgemäßen diagnostischen Testträgers. Hierzu wird Blut der Probenaufgabeseite des Testfeldes zugeführt und die Nachweisseite auf eine Farbbildung hin beobachtet, wobei die Intensität der Farbbildung ein Maß für die Menge an Analyt in der untersuchten Blutprobe darstellt.

Die Filmschichten des erfindungsgemäßen diagnostischen Testträgers werden aus Dispersionen oder Emulsionen polymerer Filmbildner hergestellt. Dispersionsfilmbildner enthalten mikroskopische, in der Trägerflüssigkeit (meist Wasser) unlösliche Polymerteilchen, welche in feinster Verteilung in der Trägerflüssigkeit dispergiert sind. Wird bei der Filmbildung die Flüssigkeit durch Verdampfen entfernt, so nähern sich die Teilchen und berühren sich schließlich. Durch die dabei auftretenden großen Kräfte und einen mit der Filmbildung einhergehenden Gewinn an Oberflächenenergie wachsen die Teilchen zu einer weitgehend geschlossenen Filmschicht zusammen. Alternativ kann auch eine Emulsion des Filmbildners verwendet werden, bei der dieser in einem Lösungsmittel gelöst ist. Das gelöste Polymer ist in einer Trägerflüssigkeit emulgiert, die mit dem Lösungsmittel nicht mischbar ist.

Als Polymere für solche Filmbildner eignen sich insbesondere Polyvinylester, Polyvinylacetate, Polyacrylester, Polymethacrylsäure, Polyvinylamide, Polyamide und Polystyrol. Neben Homopolymeren sind auch Mischpolymerisate, z. B. von Butadien, Styrol oder Maleinsäureester geeignet.

In dem Testfeld des erfindungsgemäßen diagnostischen Testträgers befinden sich die zwei genannten Filmschichten auf einer transparenten Folie. Hierfür kommen insbesondere solche Kunststoffolien in Betracht, die flüssigkeitsundurchlässig sind. Polycarbonatfolie hat sich als besonders bevorzugt erwiesen.

Die beiden Filmschichten können aus Beschichtungsmassen hergestellt werden, die den gleichen polymeren Filmbildner enthalten oder sie können aus Beschichtungsmassen erzeugt werden, die unterschiedliche polymere Filmbildner enthalten.

Während die erste Schicht ein Quellmittel und gegebenenfalls einen schwach lichtstreuenden Füllstoff enthält, benötigt die zweite Schicht ein Quellmittel und in jedem Fall wenigstens ein stark lichtsstreuendes Pigment. Daneben kann die zweite Schicht auch nicht-poröse Füllstoffe sowie poröse Füllstoffe, wie Kieselgur in geringen Mengen enthalten, ohne dadurch für Erythrozyten durchlässig zu werden. Das Gewichtsverhältnis von Pigment zu Kieselgur sollte mindestens 2:1 betragen.

Durch Zugabe eines gut quellenden Quellmittels (das heißt, einer Substanz, die unter Aufnahme von Wasser ihr Volumen vergrößert) erhält man nicht nur Schichten, die relativ schnell von Probenflüssigkeit penetriert werden, sondern die trotz dieser Öffnungswirkung des Quellmittels gute Erythrozyten- und außerdem auch Blutfarbstoffabtrenneigenschaften besitzen. Die Quelleigenschaften sollten so gut sein, daß für einen Test, bei dem die Geschwindigkeit der Farbbildung - wie beispielsweise einer Glucosenachweisreaktion - überwiegend von der Penetration der Probenflüssigkeit durch die Schicht abhängt, die optisch nachweisbare Reaktion nach maximal einer Minute meßbar ist. Als besonders geeignete Quellmittel haben sich Methylvinylethermaleinsäureanhydrid-Copolymer, Xanthangum und Methylvinylethermaleinsäure-Copolymer erwiesen.

Kieselgur wird auch als Diatomeenerde bezeichnet. Es handelt sich um aus den Kieselsäuregerüsten der Diatomeenarten entstandene Ablagerungen, die an verschiedenen Orten abgebaut werden. Die bevorzugt eingesetzte Kieselgur hat einen mittleren Teilchendurchmesser von 5-15 µm, wobei diese Werte mit einem Laser-Granulometer Typ 715 bestimmt wurden, welches von der Firma Pabisch, München, Bundesrepublik Deutschland, vertrieben wird.

Der stark lichtstreuende Pigmentanteil der zweiten Schicht liegt bei mindestens 25 Gewichts-%, bezogen auf die getrocknete und einsatzbereite Doppelschicht des Testfeldes. Da die schwach lichtstreuenden Füllstoffe und die stark lichtstreuenden Pigmente wesentlich für die optischen Eigenschaften der Filmschichten verantwortlich sind, besitzen die erste und die zweite Filmschicht unterschiedliche Füllstoffe und Pigmente.

Die erste Filmschicht soll entweder keine oder solche Füllstoffe enthalten, deren Brechungsindex nahe beim Brechungsindex von Wasser liegt. Als besonders geeignet hierfür haben sich Siliziumdioxid, Silikate und Aluminiumsilikate erwiesen. Ein Natriumaluminiumsilikat mit dem Handelsnamen Transpafill® ist besonders bevorzugt. Es besitzt eine durchschnittliche Zusammensetzung von 66 Gew.-% SiO₂, 26 Gew.-% Al₂O₃, 7 Gew.-% Na₂O und 1 Gew.-% SO₃. Die mittlere Korngröße besonders bevorzugter Primärteilchen beträgt etwa 0,06 µm.

Die zweite Schicht soll erfindungsgemäß sehr stark lichtstreuend sein. Idealerweise liegt der Brechungsindex der Pigmente in der zweiten Filmschicht mindestens bei 2,5. Daher wird vorzugsweise Titandioxid eingesetzt. Teilchen mit einem mittleren Durchmesser von etwa 0,2 bis 0,8 µm haben sich als besonders vorteilhaft erwiesen. Leicht verarbeitbare Titandioxid-Typen in der Anatas-Modifikation sind ganz besonders bevorzugt.

Reagenzsysteme zum Nachweis bestimmter Analyte durch Farbbildung sind dem Fachmann bekannt. Es ist möglich, daß sich sämtliche Komponenten des Reagenzsystems in einer Filmschicht befinden. Es ist aber auch möglich, daß die Komponenten des Reagenzsystems auf beide Filmschichten verteilt sind. Vorteilhafterweise befindet sich das farbbildende Reagenzsystem wenigstens zum Teil in der ersten Filmschicht.

Unter Farbbildung wird im Rahmen der vorliegenden Erfindung nicht nur der Übergang von weiß nach farbig verstanden, sondern auch jede Farbveränderung, wobei natürlich solche Farbveränderungen besonders bevorzugt sind, die mit einer möglichst großen Verschiebung der maximalen Absorptionswellenlinie (λ ₘₐₓ) einhergehen.

Für die Optimierung des Testfeldes in dem erfindungsgemäßen diagnostischen Testträger hat es sich als besonders vorteilhaft erwiesen, wenn beide Filmschichten ein nicht hämolysierendes Netzmittel enthalten. Neutrale, das heißt, nicht geladene Netzmittel sind hierfür besonders geeignet. Ganz besonders bevorzugt wird N-Octanoyl-N-methyl-glucamid.

Zur Herstellung eines Testfeldes eines erfindungsgemäßen diagnostischen Testträgers werden die jeweiligen Filmschichten jeweils nacheinander aus einer homogenen Dispersion der genannten Bestandteile hergestellt. Hierzu verwendet man als Unterlage für das Ausformen der Beschichtungsmasse für die erste Filmschicht die transparente Folie. Nach Aufbringen der Beschichtungsmasse für die erste Filmschicht in einer bestimmten Schichtdicke wird die Schicht getrocknet. Danach wird auf diese Schicht die Beschichtungsmasse für die zweite Schicht ebenfalls in einer dünnen Schichtdicke aufgebracht und getrocknet. Nach dem Trocknen sollte die Dicke der ersten und zweiten Filmschicht zusammen maximal 0,20 mm, bevorzugt maximal 0,12 mm, besonders bevorzugt maximal 0,08 mm betragen. Vorzugsweise ist die trockene zweite Filmschicht etwa 2 bis 5 mal dicker als die erste.

Das so hergestellte Testfeld kann zur besseren Handhabung auf einer Tragschicht befestigt sein, wobei für eine solche Schicht insbesondere solche Materialien in Frage kommen, die die zu untersuchende Flüssigkeit nicht aufnehmen. Dies sind sogenannte nicht-saugfähige Materialien, wobei Kunststoffolien beispielsweise aus Polystyrol, Polyvinylchlorid, Polyester, Polycarbonat oder Polyamid besonders bevorzugt sind. Es ist jedoch auch möglich, saugfähige Materialien, wie zum Beispiel Holz, Papier oder Pappe mit wasserabstoßenden Mitteln zu imprägnieren oder mit einem wasserfesten Film zu überziehen, wobei als Hydrophobierungsmittel Silikone oder Hartfette und als Filmbildner beispielsweise Nitrocellulose oder Celluloseacetate verwendet werden können. Als weitere Trägermaterialien eignen sich Metallfolien oder Glas.

Zur Bestimmung des in der Probenflüssigkeit, im vorliegenden Fall vorzugsweise Vollblut, aber natürlich können auch von Blut abgeleitete Proben wie Plasma oder Serum oder auch andere wässrige Flüssigkeiten untersucht werden, nachzuweisenden Analyts muß in dem erfindungsgemäßen diagnostischen Testträger die Nachweisseite des Testfeldes, die auf Farbbildung hin beobachtet und vermessen wird, durch die Tragschicht sichtbar sein. Dies kann dadurch erreicht werden, daß die Tragschicht transparent ist. Es ist aber auch möglich, daß die Tragschicht eine Lochung aufweist, die von der Nachweisseite des Testfeldes überdeckt ist. Durch die Lochung ist dann diese Nachweisseite sichtbar. In einer bevorzugten Ausführungsform des erfindungsgemäßen diagnostischen Testtragers befindet sich in der Tragschicht unterhalb der Nachweisseite des Testfeldes ein Loch, durch das die Nachweisseite des Testfeldes beobachtbar ist. Das Loch besitzt einen etwas kleineren Durchmesser als die kleinste Längenausdehnung des Testfeldes, so daß das Testfeld außerhalb des Loches auf der Tragschicht aufliegt und dort befestigt sein kann.

Aufgrund der Konstruktion des Testfeldes, insbesondere der Eigenschaft beider Filmschichten, Erythrozyten nicht auf die Nachweisseite durchdringen zu lassen, werden für die Bestimmung eines Analyts nur sehr geringe Volumina benötigt. Bei einer Größe des Testfeldes von 5 x 6 mm reichen beispielsweise bereits 3 µl Vollblut zur Bestimmung von Glucose in dieser Flüssigkeit. Um bei der Verwendung größerer Probenvolumina von etwa 15 - 20 µl ebenfalls zuverlässig arbeiten zu können und ein Herauslaufen von Flüssigkeit aus dem Testträger zu verhindern, hat sich der Einbau des Testfeldes in einen diagnostischen Testträger als besonders geeignet erwiesen, bei dem dieser Testträger eine Tragschicht mit einem darauf angeordneten Testfeld beinhaltet und das Testfeld von einem Netzwerk überdeckt ist, das größer als das Testfeld ist und das außerhalb des Testfeldes auf der Tragschicht befestigt ist. Das Netzwerk eines solchen besonders bevorzugten erfindungsgemäßen diagnostischen Testträgers ist hydrophil, aber alleine nicht kapillaraktiv. Über den Bereichen des Netzwerkes, die über das Testfeld hinausragen, d. h. den Bereichen des Netzwerkes, die nicht auf dem Testfeld aufliegen, ist eine inerte Abdeckung aus probenundurchlässigem Material so angeordnet, daß auf dem Bereich des Netzwerkes, der über dem Testfeld liegt, eine Fläche zur Probenaufgabe frei bleibt.

Das Netzwerk dieses besonders bevorzugten erfindungsgemäßen diagnostischen Testträgers soll selbst nicht kapillaraktiv oder saugfähig sein, damit die Probenflüssigkeit möglichst vollständig für das Testfeld zur Verfügung steht. Als geeignet haben sich solche Netzwerke erwiesen, die beim senkrechten Eintauchen in Wasser eine Steighöhe des Wassers im Netzwerk von weniger als 2 mm ermöglichen. Vorzugsweise werden als Netzwerk grobmaschige monofile Gewebe eingesetzt, die hydrophil sind. Hierfür kann das Gewebematerial selbst hydrophil sein oder es kann, beispielsweise durch Behandlung mit Netzmittel, hydrophil gemacht werden. Als besonders bevorzugtes Netzmaterial wird Polyester verwendet, wobei das Netz aus diesem Material dann mit Netzmittel behandelt eingesetzt wird.

Die Dicke des Netzwerkes muß so beschaffen sein, daß die darauf liegende Abdeckung und die darunterliegende Schicht sich in einem solchen Abstand voneinander befinden, daß verbleibende Flüssigkeit über dem gesättigten Testfeld und den gefüllten Maschen des Netzwerkes durch Kapillarkraft in den Bereich unter der Abdeckung aufgesaugt und von der Probenauftragsstelle weggeführt wird. In der Regel ist hierfür eine Dicke des Netzwerkes von 50 - 400 µm vorteilhaft.

Das Netz muß eine ausreichend große Maschenweite aufweisen, damit Flüssigkeit durch das Netz auf das Testfeld gelangt. Aufgrund der Beschaffenheit des Netzwerkes wird Flüssigkeit nicht im Netz über die Netzoberfläche horizontal gespreitet, sondern sie fließt vertikal durch das Netz auf das Testfeld.

In dem vorstehend beschriebenen besonders bevorzugten erfindungsgemäßen diagnostischen Testträger ist das das Testfeld überdeckende Netzwerk größer als das darunter liegende Testfeld. Der über das Testfeld hinausragende Teil des Netzwerkes ist auf der Tragschicht befestigt. Die Befestigung kann nach dem Fachmann aus der Testträgertechnologie bekannten Methoden erfolgen. Beispielsweise kann die Befestigung mittels Schmelzkleber oder härtendem Kaltkleber erfolgen. Als vorteilhaft haben sich auch doppelseitig klebende Streifen erwiesen. In allen Fällen ist es jedoch wichtig, daß die Befestigung des Netzwerkes auf der Tragschicht so erfolgt, daß von dem Testfeld ein kapillaraktiver Flüssigkeitstransport in den Teil des Netzwerkes möglich ist, der auf der Tragschicht befestigt ist. Dieser kapillaraktive Flüssigkeitstransport muß insbesondere dann möglich sein, wenn das Testfeld mit Flüssigkeit gesättigt ist. Für die Verarbeitung ganz besonders geeignet hat sich Klebeband mit Natur- oder Synthesekautschuk erwiesen. Ganz besonders vorteilhaft ist es, wenn das Mittel, das zur Befestigung des Netzwerkes auf der Tragschicht dient, etwa die gleiche Dicke wie das Testfeld hat. Es dient dann quasi als Abstandshalter, um das Netzwerk auch außerhalb des Bereiches des Testfeldes insgesamt auf einer durchgehenden Fläche eben zu halten.

Über dem Netzwerk des besonders bevorzugten erfindungsgemäßen diagnostischen Testträgers ist eine inerte Abdeckung aus probenundurchlässigem, in der Regel wasserundurchlässigem und nicht saugfähigem Material, so angeordnet, daß der Bereich des Netzwerkes außerhalb des Testfeldes abgedeckt ist. Idealerweise ragt die Abdeckung auch noch ein wenig über den Bereich des Testfeldes. Aufjeden Fall bleibt jedoch ein erheblicher Teil des Netzwerkes, das das Testfeld bedeckt, frei. Dieser freie Teil des Netzwerkes wird als Probenauftragsstelle bezeichnet.

Als Abdeckung haben sich Kunstoffolien als besonders vorteilhaft erwiesen. Wenn Abdeckung und Netzwerk unterschiedliche Farben haben, beispielsweise weiß und gelb oder weiß und rot, kann damit die Stelle sehr gut kenntlich gemacht werden, auf die zu untersuchende Probenflüssigkeit aufgegeben werden soll.

Auf der Abdeckung kann auch beispielsweise mit einem oder mehreren aufgedruckten Pfeilen verdeutlicht werden, in welcher Richtung, das heißt, mit welchem Ende ein erfindungsgemäßer diagnostischer Testträger in ein Meßgerät gelegt oder geschoben werden soll.

Eine Probenauftragsstelle kann besonders einfach durch eine Abdeckung mittels zweier bandförmiger Kunststoffolien erreicht werden, die einen bandartigen Bereich des das Testfeld überdeckenden Netzwerkes freilassen. Die zur Abdeckung verwendeten Folien sind auf dem Netzwerk und gegebenenfalls auf der Tragschicht befestigt. Für eine solche Befestigung eignen sich Schmelzkleber oder Klebebänder, wenn die Folien nicht selbst klebefähig sind. Auf jeden Fall ist jedoch darauf zu achten, daß unter der Abdeckung, durch das Netzwerk gebildet, ein Kapillarspalt verbleibt, in den überschüssige Probenflüssigkeit von einem mit Flüssigkeit gesättigten Testfeld aufgenommen werden kann. Die Probenauftragsstelle befindet sich vorzugsweise über der Lochung in der Tragschicht, durch die eine Farbbildung im Testfeld beobachtet werden kann.

Zur Durchführung eines Verfahrens zur Bestimmung eines Analyten aus Vollblut mit Hilfe eines erfindungsgemäßen diagnostischen Testträgers wird Blut der Probenaufgabeseite des Testfeldes zugeführt und die Nachweisseite auf eine Farbbildung hin beobachtet. Eine häufige Ursache für falsche Meßwerte beim Diabetes-Monitoring, das heißt, der regelmäßigen Kontrolle des Blutes Diabeteskranker auf den Gehalt an Glucose, ist bisher ein zu geringes Probenvolumen gewesen. Für den erfindungsgemäßen diagnostischen Testträger reichen bei einer Testfeldgröße von etwa 5x6 mm bereits 3 µl Vollblut, um eine visuelle Beurteilung des Untersuchungsergebnisses vornehmen zu können. Dadurch, daß die zweite Filmschicht des Testfeldes hoch lichtstreuend, die erste Schicht dagegen schwach lichtstreuend ist, wird gebildete Farbe von der Nachweisseite aus mit relativ hoher Brillianz und Farbintensität wahrgenommen. Diese ermöglicht genaue Bestimmungen trotz der durch kleine Probenvolumina bedingten kleinen Analytmengen.

Natürlich kann das Verfahren zur Bestimmung eines Analyten aus Vollblut mit Hilfe eines erfindungsgemäßen diagnostischen Testträgers auch apparativ erfolgen. Zur Erzielung möglichst genauer quantitativer Ergebnisse wird sich ein solches Verfahren auch empfehlen. Dafür wird vorzugsweise die Reflektion der Nachweisseite kontinuierlich oder in Intervallen auf eine Farbbildung hin beobachtet. In Form einer Messreihe werden Reflektionsmessungen der Nachweisseite des Testfeldes vorgenommen. Nach ein- oder mehrmaligem Unterschreiten eines bestimmten Differenzwertes aufeinander folgender Messungen und gegebenenfalls einer weiteren fixen Reaktionszeit wird die Messung beendet. Der letzte Reflektionswert der Meßreihe wird dann zur mathematischen Auswertung der Analytkorzentration verwendet.

Da dieses Verfahren mit einem eindeutigen Kriterium zum Abbruch der Meßreihe arbeitet und den Zeitpunkt des Probenauftrags nicht benötigt, kann der Probenauftrag auch außerhalb des Meßgerates stattfinden. Dabei genügt es, den Streifen zu einem beliebigen Zeitpunkt innerhalb einer maximal zulässigen Zeitspanne nach Probenauftrag in das Gerät einzulegen.

Im vorliegenden Fall ist das Bestimmungsverfahren im Bereich von Hämatokritwerten von 20 bis mehr als 60 % unbeeinflußt und kann deshalb als hämatokritunabhängig bezeichnet werden, wenn die Bestimmung apparativ nach dem vorstehenden Verfahren durchgeführt wird. Für die visuelle Bestimmung muß 2-3 Minuten gewartet werden, bevor ein hämatokrit-unabhängiger Wert abgelesen werden kann.

In Fig. 1-9 ist die Erfindung schematisch dargestellt.

Fig. 1 zeigt einen Querschnitt durch das Testfeld eines erfindungsgemäßen diagnostischen Testträgers.

Fig. 2 zeigt eine perspektivische Ansicht eines besonders bevorzugten erfindungsgemäßen diagnostischen Testträgers.

Fig. 3 zeigt eine Aufsicht auf die Unterseite eines erfindungsgemäßen diagnostischen Testträgers gemäß Fig. 2.

Fig. 4 zeigt einen Querschnitt durch einen erfindungsgemäßen diagnostischen Testträger gemäß Fig. 2 entlang A-A.

Fig. 5 zeigt eine Vergrößerung eines Teils des Querschnitts aus Fig. 4.

Fig. 6 - 9 zeigen Kalibrationskurven 1 - 4, deren Erzeugung in Beispiel 2 näher erläutert wird.

Die in den Figuren verwendeten Bezugszeichen lauten:
- 1: Testfeld
- 2: transparente Folie
- 3: erste Schicht
- 4: zweite Schicht
- 5: Probenaufgabeseite
- 6: Nachweisseite
- 7: diagnostischer Testtrager
- 8: Tragschicht
- 9: Netzwerk
- 10: Abdeckung
- 11: über Testfeld hinausragender Bereich des Netzwerkes
- 12: Probenauftragsstelle
- 13: Lochung
- 14: Klebeband für Testfeld
- 15: Abstandhalter
- 16: kapillaraktiver Spalt
- 17: Probenflüssigkeit
- 18: Positionierloch

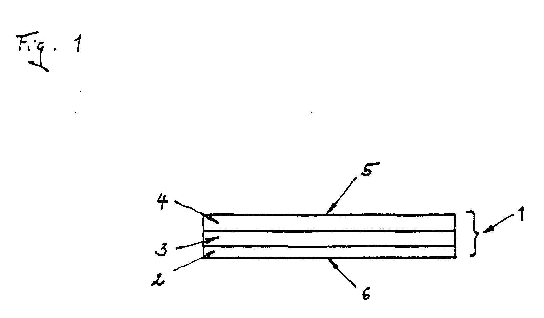

Der in Fig. 1 dargestellte Querschnitt durch das Testfeld (1) eines erfindungsgemäßen diagnostischen Testträgers zeigt die transparente Folie (2), auf die die erste Filmschicht (3) aufgebracht ist. Die erste Filmschicht (3) ist überdeckt durch die zweite Filmschicht (4). Durch die Herstellungsweise des Testfeldes (1) des erfindungsgemäßen Testträgers, nämlich Beschichtung der transparenten Folie (2) mit einer feuchten Beschichtungsmasse für die erste Filmschicht (3) und nachfolgende Beschichtung der getrockneten ersten Filmschicht (3) mit einer feuchten Beschichtungsmasse für die zweite Filmschicht (4) ergibt sich, daß die Schichten untereinander und die erste Filmschicht (3) auf der transparenten Folie (2) vollflächig miteinander haftend verbunden sind. Blut, das auf Inhaltsstoffe untersucht werden soll, wird auf die Probenaufgabeseite (5) des Testfeldes (1) des erfindungsgemäßen Testträgers aufgegeben. Bei Anwesenheit von Analyt in der Probe wird von der Nachweisseite (6) des Testfeldes (1) des erfindungsgemäßen Testträgers eine Farbbildung beobachtbar sein, deren Intensität von der Menge an Analyt in der Probe abhängig ist.

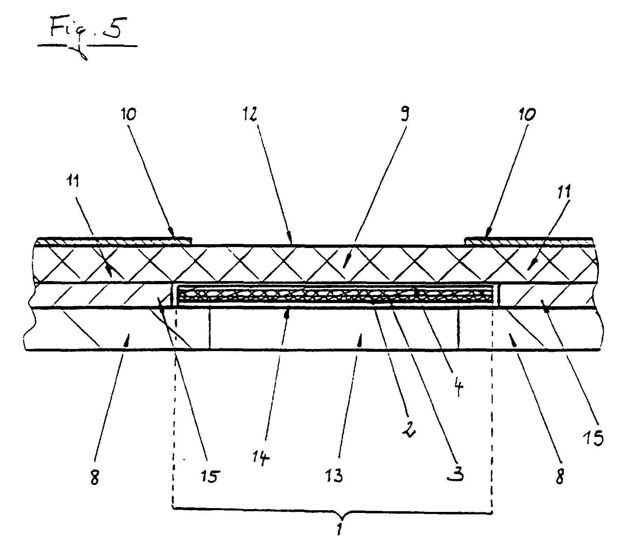

Der in Fig. 2 perspektivisch und in Fig. 4 im Querschnitt gezeigte besonders bevorzugte erfindungsgemäße diagnostische Testträger (7) besitzt die Form eines Teststreifens. Auf einer Tragschicht (8) befindet sich das Testfeld (1), das durch ein größeres Netzwerk (9) überdeckt ist. Neben dem Testfeld (1) ist das Netzwerk (9) mittels Abstandhalter (15) auf der Tragschicht (8) befestigt. Diese Abstandhalter (15) können Schmelzkleberflächen oder zweiseitig klebende Bänder sein, die das Netzwerk (9) auf der Tragschicht (8) fixieren. Idealerweise besitzen die Abstandhalter (15) in etwa die gleiche Dicke wie das Testfeld (1). Die als Abdeckung (10) dienenden Schichten sind auf der Tragschicht (8) und dem Netzwerk (9) befestigt. Sie sind so angeordnet, daß sie den über das Testfeld (1) hinausragenden Bereich des Netzwerkes (9) überdecken. Geringfügig ragen die Abdeckungen (10) auch noch über das Testfeld (1). Sie lassen jedoch den größten Teil des Netzwerkes (9) frei, der das Testfeld (1) überdeckt. Dieser Bereich stellt die Probenauftragsstelle (12) dar. Hierauf wird die zu untersuchende Probenflüssigkeit (17) aufgegeben. Das Positionierloch (18) dient dazu, den Teststreifen im Falle einer apparativen, beispielsweise einer reflektionsphotometrischen Vermessung, an einer genau vorbestimmten Stelle des Apparates festzuhalten. Dies kann dadurch geschehen, daß beispielsweise ein Stift in das Positionierloch (18) hineinragt und so den Testträger (7) an einer vorbestimmten Stelle festhält. Die linke Abdeckung (10) enthält aufgedruckt Pfeile, die dem Anwender zeigen, mit welchem Ende der Testträger (7) in ein Meßgerät gelegt oder geschoben werden soll.

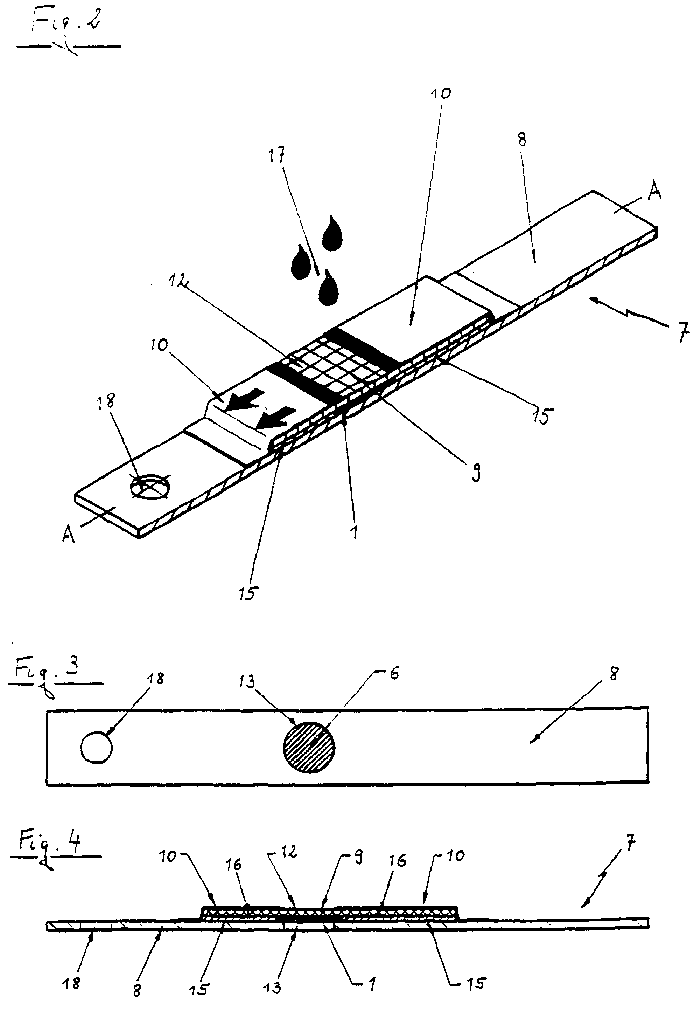

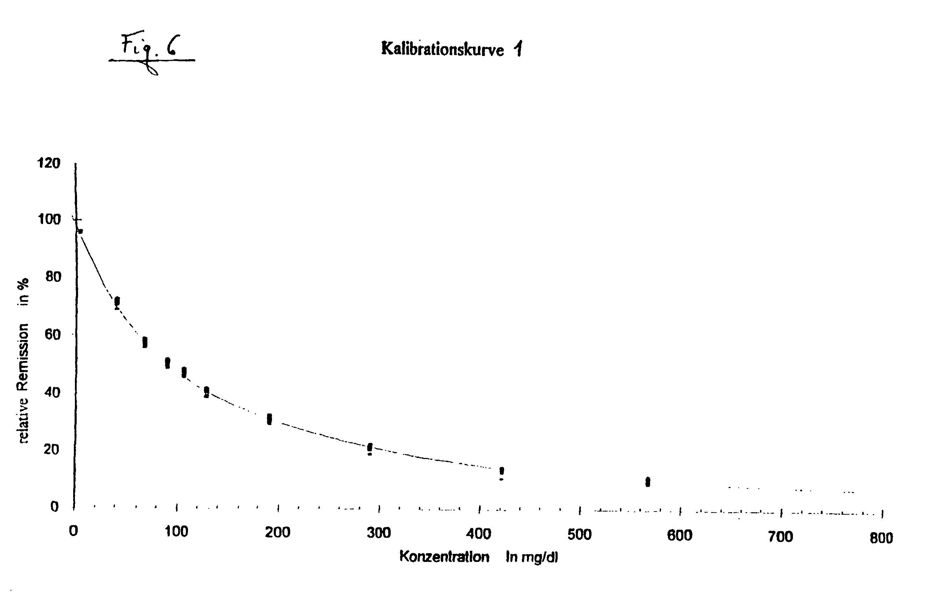

Fig. 5 zeigt einen vergrößerten Querschnitt durch einen besonders bevorzugten erfindungsgemäßen diagnostischen Testträger, wie er in Fig. 2 und 4 dargestellt ist. An dieser Figur soll erläutert werden, wie ein Verfahren zur Bestimmung eines Analyts in einer flüssigen Probe, beispielsweise Glucose in Vollblut, abläuft. Für eine solche Bestimmung wird Blut auf die Probenaufgabestelle (12) des Netzwerkes (9) gegeben. Die Flüssigkeit gelangt vertikal durch das Netzwerk (9) auf das Testfeld (1). Dort werden im Falle von Blut als Probenflüssigkeit Erythrozyten zurückgehalten, während Plasma oder Serum in die Schichten (3,4) des Testfeldes (1) gelangt. Sofern ausreichend Probenflüssigkeit in Form von Vollblut aufgegeben wurde, verteilt sich Plasma oder Serum im Testfeld (1) in der ersten und zweiten Filmschicht (3,4) über die gesamte Fläche des Testfeldes (1). Bei sehr kleinen Flüssigkeitsvolumina kann das Testfeld (1) das darüberliegende Netzwerk (9) sogar trockensaugen, da das Netzwerk (9) nicht selbst kapillaraktiv ist. Bei mittleren bis großen Flüssigkeitsvolumina füllen sich zunächst die Hohlräume des Netzwerkes (9) über dem Testfeld (1) und anschließend die kapillaren Hohlräume unter den Abdeckungen (10). Für die ordnungsgemäße Funktion dieser kapillaren Hohlräume ist es notwendig, daß die Abdeckungen (10) wenigstens etwas mit dem Bereich des Testfeldes (1) unter dem Netzwerk (9) überlappen. Durch die Lochung (13) kann die Nachweisseite des Testfeldes (1) beobachtet werden. Für diesen Aspekt ist in Fig. 3 eine Aufsicht auf die Unterseite des diagnostischen Testträgers gemäß Fig. 2,4 und 5 dargestellt. Im Falle der Anwesenheit von Analyt in der aufgebrachten Probenflüssigkeit wird sich die Farbe der Nachweisseite (6) des Testfeldes (1) verändern. Es bildet sich eine Farbe, deren Intensität ein Maß für die Menge an Analyt in der Probenflüssigkeit ist.

Die Erfindung wird durch die nachfolgenden Beispiele noch weiter erläutert.

### Beispiel 1

### Herstellung eines erfindungsgemäßen diagnostischen Testträgers

Die Herstellung eines Testtragers gemäß Fig. 2 erfolgt mit folgenden Arbeitsschritten:

Auf eine Titandioxid-haltige Polyester-Tragschicht wird ein 5 mm breites doppelseitiges Klebeband (Polyesterträger und Synthesekautschuk-Kleber) aufgebracht. Dieser Verbund wird mit einem Lochabstand von 6 mm gemeinsam gestanzt, um die Meßlöcher zu erzeugen. Danach wird das Schutzpapier des doppelseitigen Klebebands abgezogen.

Zur Herstellung eines Testfeldes, das aus 2 Filmschichten aufgebaut ist, wird so vorgegangen:
A. In einem Becherglas werden die folgenden Komponenten als Reinsubstanzen oder in Form von Stammlösungen in folgender Zusammensetzung zusammengegeben und durch Rühren gemischt:

| | |
|---|---|
| Wasser | 820,0 g |
| Citronensäure-1-hydrat | 2,5 g |
| Calciumchlorid-2-hydrat | 0,5 g |
| Natriumhydroxid | 1,4 g |
| Xanthan gum | 3,4 g |
| Tetraethylammoniumchlorid | 2,0 g |
| N-Octanoyl-N-methyl-glucamid | 2,1 g |
| Polyvinylpyrrolidon (MG 25000) | 3,5 g |
| Transpafill® (Natrium-Aluminiumsilikat) | 62,1 g |
| Polyvinylpropionat-Dispersion (50 Gew.-% in Wasser) | 60,8 g |
| Bis-(2-hydroxyethyl)-(4-hydroximinocyclohexa-2,5-dienylidin)-ammoniumchlorid | 1,2 g |
| 2,18-Phosphormolybdänsäure-hexanatriumsalz | 16,1 g |
| Pyrrolochinolin-chinon | 32 mg |
| Glucosedehydrogenase rec. aus Acinetobacter calcoaceticus, | 1,7 MU |
| EC 1.1.99.17 | (2,4 g) |
| 1-Hexanol | 1,6 g |
| 1-Methoxy-2-propanol | 20,4 g |

Die Gesamtmasse wird mit NaOH auf einen pH von ca. 6 eingestellt und dann mit einem Flächengewicht von 89 g/qm auf eine 125 µ dicke Polycarbonatfolie aufgetragen und getrocknet.
B. In einem Becherglas werden die folgenden Komponenten als Reinsubstanzen oder in Form von Stammlösungen in folgender Zusammensetzung zusammengegeben und durch Rühren gemischt:

| | |
|---|---|
| Wasser | 579,7 g |
| Natriumhydroxid | 3,4 g |
| Gantrez® (Methylvinylether-maleinsaure-Copolymer) | 13,8 g |
| N-Octanoyl-N-methyl-glucamid | 3,6 g |
| Tetraethylammoniumchlorid | 9,7 g |
| Polyvinylpyrrolidon (MG 25000) | 20,2 g |
| Titandioxid | 177,1 g |
| Kieselgur | 55,3 g |
| Polyvinylpropionat-Dispersion (50 Gew.-% in Wasser | 70,6 g |
| 2,18-Phosphormolybdänsäure-hexanatriumsalz | 44,3 g |
| Kaliumhexacyanoferrat (III) | 0,3 g |
| 1-Hexanol | 1,6 g |
| 1-Methoxy-2-propanol | 20,4 g |

Die Gesamtmasse wird mit NaOH auf einen pH von ca. 6 eingestellt und dann mit einem Flächengewicht von 104 g/qm auf die wie vorstehend unter A. beschriebene beschichtete Polycarbonatfolie aufgetragen und getrocknet.

Ein 5 mm breiter Streifen der so hergestellten Nachweisschicht wird mit der Folienseite auf das gestanzte doppelseitige Klebeband auf der Tragschicht passgenau aufgeklebt.

Direkt an die Nachweisschicht angrenzend werden auf beiden Seiten doppelseitige Klebebänder (PVC-Träger und Naturkautschuk-Kleber) als Abstandshalter auf die Trägerfolie aufgeklebt. Im vorliegenden Beispiel ist ein Abstandshalter 6 mm und der andere 9 mm breit. Danach wird die Schutzfolie der beiden doppelseitigen Klebebänder abgezogen.

Auf diesen Verbund wird ein mit Netzmittel imprägniertes gelbes monofiles grobmaschiges Polyestergewebe Scrynel PE 280 HC (Züricher Beuteltuchfabrik, Rüschlikon, Schweiz) aufgelegt und durch Anpressen verklebt.

Es werden zwei einseitige Klebebänder (PVC-Träger und Naturkautschuk-Kleber) als Abdeckungen so auf das gelbe Netz aufgeklebt, daß die Abstandshalter ganz abgedeckt werden und wenigstens noch eine geringfügige Überlappung mit dem Reaktivbezirk stattfindet. Damit ist die Bandware fertiggestellt.

Die Bandware wird so in 6 mm breite Testträger geschnitten, daß das Meßloch im Testträger mittig liegt.

### Beispiel 2

### Hämatokritunabhängigkeit der erfindungsgemäßen Testträger

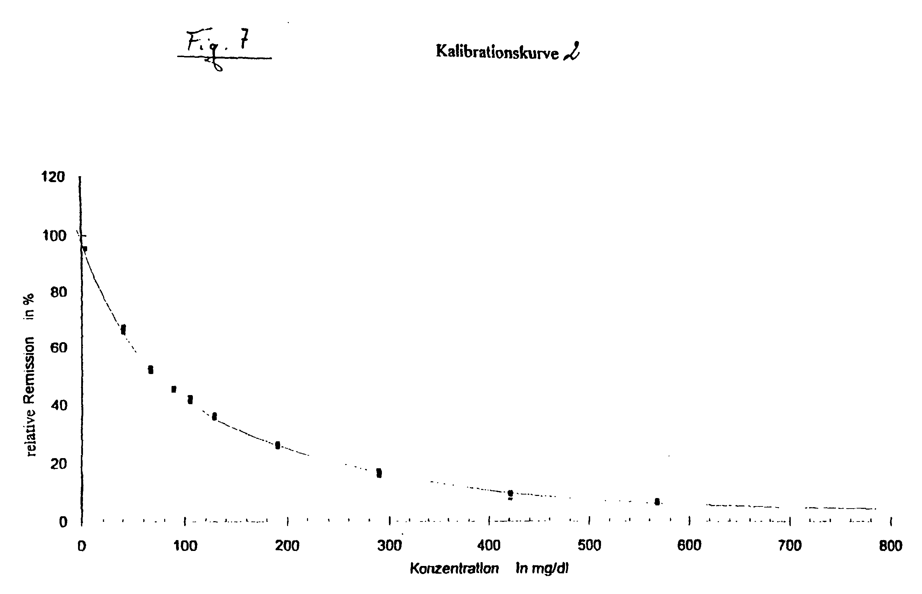

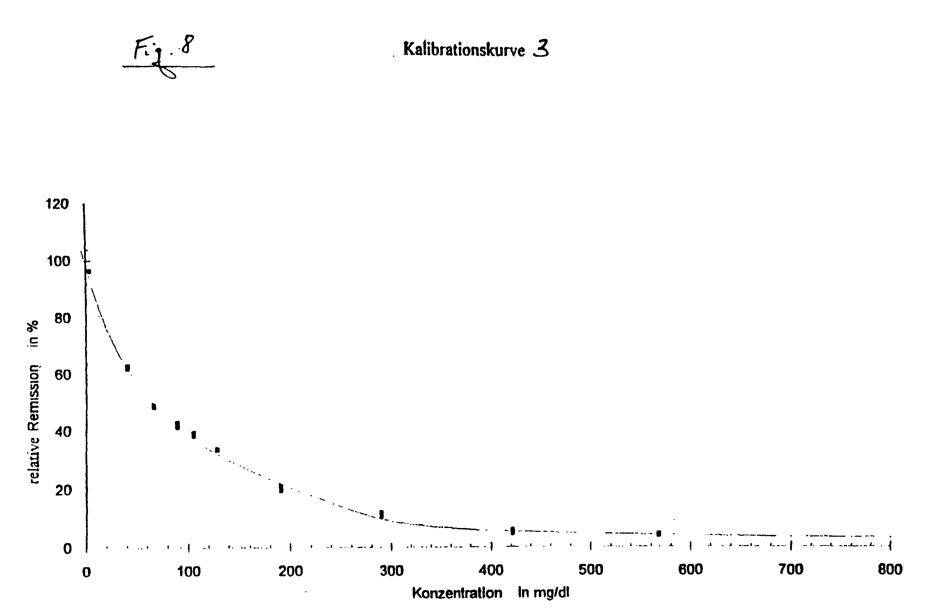

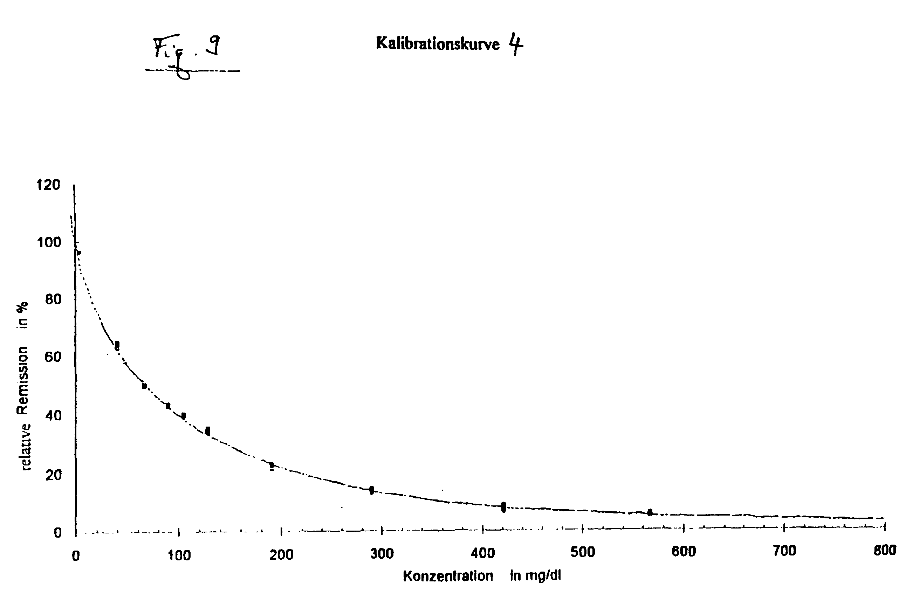

Die Testträger aus Beispiel 1 können mit einem Reflexions-photometer vermessen werden. Die Remissionswerte, die ein Maß für die Farbintensität darstellen, können bei Vorliegen einer Kalibrationskurve in Glucosekonzen-trationen umgerechnet werden. Sofern die Bezeichnung "relative Remissionen" verwendet wird, sind die Remissionen auf den trockenen Testträger bezogen.
A. Kalibrationskurven werden dadurch erzeugt, daß eine große Zahl von Venenbluten mit unterschiedlichen Glucosekonzentrationen vermessen wird. Aus den Remissionswerten und den mit einer Referenzmethode ermittelten Glucosekonzentrationen dieser Venenblute kann dann eine Kalibrationskurve erstellt werden.
Bei der Kalibrationsvariante 1 wurden 10 µl Venenblut auf Testträger gemäß Beispiel 1 aufgetragen und die Remissionen nach 21 sec erfaßt. Aus den gemittelten Remissionen von 10 Testträgern und den Referenzwerten der Blutproben wurde die Kalibrationskurve 1 (Fig. 6) durch Regressionsrechnung ermittelt.
Bei der Kalibrationsvariante 2 wurden ebenfalls 10 µl Venenblut auf Testträger gemäß Beispiel 1 aufgetragen und die Remissionen nach 30 sec erfaßt. Aus den gemittelten Remissionen von 10 Testträgem und den Referenzwerten der Blutproben wurde die Kalibrationskurve 2 (Fig. 7) durch Regressionsrechnung ermittelt.
Bei der Kalibrationsvariante 3 wurden ebenfalls 10 µl Venenblut auf Testträger gemäß Beispiel 1 aufgetragen und die Remissionen in Abständen von 3 sec erfaßt. Sobald die Remissionsdifferenzen zweimal hintereinander geringer als 0,3 waren, wurde die Messung abgebrochen und der Remissionswert zur Auswertung herangezogen. Aus den gemittelten Remissionen von 10 Testträgern und den Referenzwerten der Blutproben wurde die Kalibrationskurve 3 (Fig. 8) durch Regressionsrechnung ermittelt.
Bei der Kalibrationsvariante 4 wurden ebenfalls 10 µl Venenblut auf Testträger gemäß Beispiel 1 aufgetragen und die Remissionen in Abständen von 3 sec erfaßt. Sobald die Remissionsdifferenzen zweimal hintereinander geringer als 0,9 waren, wurde die Messung abgebrochen und der Remissionswert zur Auswertung herangezogen. Aus den gemittelten Remissionen von 10 Testträgern und den Referenzwerten der Blutproben wurde die Kalibrationskurve 4 (Fig. 9) durch Regressionsrechnung ermittelt.
B. Bei der Meßvariante 1 wurden 10 µl Venenblut auf Testträger gemäß Beispiel 1 aufgetragen und die Remissionen nach 21 sec erfaßt. Die einzelnen Remissionen wurden mit der entsprechenden Kalibrationskurve aus Fig. 6 in Glucosekonzentrationen umgerechnet. Aus den gemittelten Konzentrationen von 10 Testträgern und den Referenzwerten der Blutproben wurde die Richtigkeitsabweichung ermittelt und in Tabelle 1 dargestellt.
Bei der Meßvariante 2 wurden ebenfalls 10 µl Venenblut auf Testträger gemäß Beispiel 1 aufgetragen und die Remissionen nach 30 sec erfaßt. Die einzelnen Remissionen wurden mit der entsprechenden Kalibrationskurve gemäß Fig. 7 in Glucosekonzentrationen umgerechnet. Aus den gemittelten Konzentrationen von 10 Testträgern und den Referenzwerten der Blutproben wurde die Richtigkeitsabweichung ermittelt und in Tabelle 2 dargestellt.
Bei der erfindungsgemaßen Meßvariante 3 wurden ebenfalls 10 µl Venenblut aufTestträger gemäß Beispiel 1 aufgetragen und die Remissionen in Abständen von 3 sec erfaßt. Sobald die Remissionsdifferenzen zweimal hintereinander geringer als 0,3 waren, wurde die Messung abgebrochen und der Remissionswert zur Auswertung herangezogen. Die einzelnen Remissionen wurden mit der entsprechenden Kalibrationskurve gemäß Fig. 8 in Glucosekonzentrationen umgerechnet. Aus den gemittelten Konzentrationen von 10 Testträgern und den Referenzwerten der Blutproben wurde die Richtigkeitsabweichung ermittelt und in Tabelle 3 dargestellt.
Bei der erfindungsgemäßen Meßvariante 4 wurden ebenfalls 10 µl Venenblut aufTestträger gemäß Beispiel 1 aufgetragen und die Remissionen in Abständen von 3 sec erfaßt.
Sobald die Remissionsdifferenzen zweimal hintereinander geringer als 0,9 waren, wurde die Messung abgebrochen und der Remissionswert zur Auswertung herangezogen. Die einzelnen Remissionen wurden mit der entsprechenden Kalibrationskurve gemäß Fig. 9 in Glucosekonzentrationen umgerechnet. Aus den gemittelten Konzentrationen von 10 Testträgem und den Referenzwerten der Blutproben wurde die Richtigkeitsabweichung ermittelt und in Tabelle 4 dargestellt.

**Tabelle 1:**

| Hämatokritabhängigkeit bei Meßvariante 1 | | | | | |
|---|---|---|---|---|---|
| Blut mit 30% Hämatokrit | | | Blut mit 57% Hämatokrit | | |
| gemessene relative Remission [%] | berechnete Konzentration nach Kalibrationskurve 1 | prozentuale Abweichung vom Referenzwert | gemessene relative Remission [%] | berechnete Konzentration nach Kalibrationskurve 1 | prozentuale Abweichung vom Referenzwert |
| 50,1 | 88,5 | 7,3 | 61,6 | 56,3 | - 31,5 |
| 44,2 | 111,0 | 4,9 | 54,3 | 75,3 | - 17,1 |
| 37,8 | 143,2 | 3,9 | 45,8 | 104,4 | - 20,4 |
| 30,1 | 197,4 | 2,9 | 36,6 | 150,6 | - 20,7 |
| 21,1 | 294,7 | 4,6 | 28,3 | 213,8 | - 20,5 |

**Tabelle 2:**

| Hämatokritabhängigkeit bei Meßvariante 2 | | | | | |
|---|---|---|---|---|---|
| Blut mit 30% Hämatokrit | | | Blut mit 57% Hämatokrit | | |
| gemessene relative Remission [%] | berechnete Konzentration nach Kalibrationskurve 2 | prozentuale Abweichung vom Referenzwert | gemessene relative Remission [%] | berechnete Konzentration nach Kalibrationskurve 2 | prozentuale Abweichung vom Referenzwert |
| 45,6 | 85,8 | 4,0 | 54,8 | 60,5 | - 25,6 |
| 39,7 | 107,6 | 1,6 | 48,3 | 77,6 | - 24,5 |
| 33,6 | 137,2 | - 0,5 | 39,0 | 110,4 | - 15,8 |
| 25,4 | 193,4 | 0,9 | 30,0 | 159,1 | - 16,2 |
| 16,3 | 295,9 | 5,0 | 22,0 | 226,3 | - 15,9 |

**Tabelle 3:**

| Kompensation der Hämatokritabhängigkeit durch erfindungsgemäße Meßvariante 3 | | | | | |
|---|---|---|---|---|---|
| Blut mit 30% Hämatokrit | | | Blut mit 57% Hämatokrit | | |
| gemessene relative Remission [%] | berechnete Konzentration nach Kalibrationskurve 3 | prozentuale Abweichung vom Referenzwert | gemessene relative Remission [%] | berechnete Konzentration nach Kalibrationskurve 3 | prozentuale Abweichung vom Referenzwert |
| 43,3 | 82,3 | - 0,2 | 46,7 | 72,4 | 1,0 |
| 37,5 | 102,6 | - 3,0 | 40,6 | 91,3 | 0,6 |
| 30,6 | 134,1 | - 2,7 | 29,6 | 139,4 | 6,2 |
| 10,8 | 274,6 | -2,5 | 11,7 | 265,8 | - 1,2 |

**Tabelle 4:**

| Kompensation der Hämatokritabhängigkeit durch erfindungsgemäße Meßvariante 4 | | | | | |
|---|---|---|---|---|---|
| Blut mit 30% Hämatokrit | | | Blut mit 57% Hämatokrit | | |
| gemessene relative Remission [%] | berechnete Konzentration nach Kalibrationskurve 4 | prozentuale Abweichung vom Referenzwert | gemessene relative Remission [%] | berechnete Konzentration nach Kalibrationskurve 4 | prozentuale Abweichung vom Referenzwert |
| 44,2 | 84,1 | 1,9 | 48,8 | 70,3 | - 2,0 |
| 38,6 | 104,6 | - 1,2 | 42,8 | 88,8 | - 2,2 |
| 32,2 | 133,6 | - 3,1 | 33,3 | 128,1 | - 2,4 |
| 22,6 | 195.5 | 2,0 | 22,8 | 194,3 | 2,3 |
| 13,5 | 296,4 | 5,2 | 15,4 | 269,7 | 0,3 |

Für 20 % Hämatokrit ergeben sich mit Meßvarianten 3 und 4 Werte mit ähnlich geringen Abweichungen wie für 30 % Hämatokrit.

## Patentansprüche

1. Diagnostischer Testträger zur Bestimmung eines Analyten aus Vollblut mit Hilfe eines in dem Träger enthaltenen Reagenzsystems, welches ein Farbbildungsreagenz einschließt, mit einem Testfeld, welches eine Probenaufgabeseite, auf die die Blutprobe zugeführt wird und eine Nachweisseite, auf der infolge der Reaktion des Analyten mit dem Reagenzsystem eine optisch nachweisbare Veränderung stattfindet, aufweist, und welches so ausgebildet ist, daß die in der Probe enthaltenen Erythrozyten:nicht auf die Nachweisseite gelangen,
wobei das Testfeld eine transparente Folie und eine erste Filmschicht und eine zweite, auf die erste Filmschicht aufgebrachte Filmschicht umfaßt,
wobei die sich auf der transparenten Folie befindliche erste Schicht im feuchten Zustand wesentlich weniger lichtstreuend ist als die darüberliegende zweite Schicht und
wobei die nicht beschichtete Seite der Folie die Nachweisseite und die Seite der zweiten Schicht, die der Seite gegenüberliegt, mit der die zweite Schicht auf der ersten aufliegt, die Probenaufgabeseite ist,
**dadurch gekennzeichnet, daß** die erste Filmschicht einen Füllstoff enthält, dessen Brechungsindex nahe beim Brechungsindex von Wasser liegt, und
daß die zweite Schicht ein Pigment mit einem Brechungsindex von mindestens 2,5 in einer Konzentration von mindestens 25 Gew.-% bezogen auf die getrocknete Doppelfilmschicht enthält.

2. Diagnostischer Testträger gemäß Patentanspruch 1, **dadurch gekennzeichnet, daß** beide Filmschichten aus einer Dispersion oder Emulsion eines polymeren Filmbildners gebildet sind, die in homogener Verteilung den polymeren Filmbildner und ein Quellmittel enthält, wobei die Quellfähigkeit des Quellmittels so hoch ist, daß die optisch nachweisbare Veränderung auf der Nachweisseite nach maximal einer Minute meßbar ist.

3. Diagnostischer Testträger gemäß einem der vorangehenden Patentansprüche, **dadurch gekennzeichnet, daß** die erste Filmschicht ein Natrium-Aluminiumsilikat als Füllstoff und die zweite Filmschicht Titandioxid als Pigment enthalten.

4. Diagnostischer Testträger gemäß einem der der vorangehenden Patentansprüche, **dadurch gekennzeichnet, daß** beide Filmschichten zur Erythrozytenabtrennung geeignet sind.

5. Diagnostischer Testträger gemäß einem der vorangehenden Patentansprüche, **dadurch gekennzeichnet, daß** die Dicke der ersten und zweiten Filmschicht im trockenen Zustand zusammen maximal 0,20 mm, bevorzugt maximal 0,12 mm, besonders bevorzugt maximal 0,08 mm beträgt.

6. Diagnostischer Testträger gemäß Patentanspruch 5, **dadurch gekennzeichnet, daß** die zweite Filmschicht etwa 2 bis 5 mal so dick ist wie die erste.

7. Diagnostischer Testträger gemäß einem der vorhergehenden Patentansprüche, **dadurch gekennzeichnet, daß** sich das farbbildende Reagenz in der ersten Filmschicht befindet.

8. Diagnostischer Testträger gemäß einem der Patentansprüche 1-6, **dadurch gekennzeichnet, daß** die Komponenten des Reagenzsystems auf beide Filmschichten verteilt sind.

9. Diagnostischer Testträger gemäß einem der vorangehenden Patentansprüche, **dadurch gekennzeichnet, daß** in den Filmschichten keine hämolysierenden Netzmittel enthalten sind.

10. Diagnostischer Testträger gemäß Patentanspruch 9, **dadurch gekennzeichnet, daß** wenigstens eine Filmschicht als Netzmittel N-Octanoyl-N-methyl-glucamid enthält.

11. Verfahren zur Bestimmung eines Analyten aus Vollblut mit Hilfe eines diagnostischen Testträgers gemäß einem der Patentansprüche 1-10, **dadurch gekennzeichnet, daß** Blut der Probenaufgabenseite des Testfeldes zugeführt und die Nachweisseite auf eine Farbbildung hin beobachtet wird, wobei die Intensität der Farbbildung ein Maß für die Menge an Analyt im Blut darstellt.

12. Verfahren gemäß Patentanspruch 11, **dadurch gekennzeichnet, daß** die Nachweisseite kontinuierlich oder in Intervallen auf eine Farbbildung hin beobachtet wird.

13. Verfahren gemäß Patentanspruch 12, **dadurch gekennzeichnet, daß** die Beobachtung apparativ in Form einer Meßreihe mittels Farbbildungsmessungen erfolgt und der Auswertezeitpunkt durch einmaliges Unterschreiten eines bestimmten absoluten oder prozentualen Differenzwertes aufeinanderfolgender Farbbildungsmessungen bestimmt wird.

14. Verfahren gemäß Patentanspruch 12, **dadurch gekennzeichnet, daß** die Beobachtung apparativ in Form einer Meßreihe mittels Farbbildungsmessungen erfolgt und der Auswertezeitpunkt durch vorher festgelegtes mehrmaliges Unterschreiten eines bestimmten absoluten oder prozentualen Differenzwertes aufeinanderfolgender Farbbildungsmessungen bestimmt wird.

15. Verfahren gemäß Patentanspruch 13 oder 14, **dadurch gekennzeichnet, daß** nach einoder mehrmaligem Unterschreiten des absoluten oder prozentualen Differenzwertes noch eine weitere Nachreaktionszeit folgt.

16. Verfahren gemäß einem der Patentansprüche 13-15, **dadurch gekennzeichnet, daß** die Farbbildungsmessungen reflektometrisch erfolgen und der letzte Meßwert der Meßreihe zur mathematischen Auswertung der Analytkonzentration verwendet wird.

## Claims

1. Diagnostic test carrier for the determination of an analyte from whole blood with the aid of a reagent system contained in the carrier which includes a colour forming reagent with a test field which has a sample application side onto which the blood sample is applied and a detection side on which an optically detectable change takes place as a result of the reaction of analyte with the reagent system and which is constructed in such a way that erythrocytes present in the sample do not reach the detection side,
the test field comprising a transparent foil and a first film layer and a second film layer applied to the first film layer,
the first layer located on the transparent foil scattering light considerably less in a wet state than the overlying second layer and
the non-coated side of the foil being the detection side and the side of the second layer which is opposite to the side of the second layer that rests on the first being the sample application side,
wherein the first film layer contains a filler which has a refractive index close to the refractive index of water and the second layer contains a pigment with a refractive index of at least 2.5 at a concentration of at least 25 % by weight based on the dried double film layer.

2. Diagnostic test carrier as claimed in claim 1, wherein both film layers are formed from a dispersion or emulsion of a polymeric film former which contains the polymeric film former and a swelling agent in homogenous dispersion, wherein the swelling capability of the swelling agent is so high that the optically detectable change can be measured on the detection side after a maximum of one minute.

3. Diagnostic test carrier as claimed in one of the previous claims, wherein the first film layer contains a sodium aluminium silicate as the filler and the second film layer contains titanium dioxide as the pigment.

4. Diagnostic test carrier as claimed in one of the previous claims, wherein both film layers are suitable for erythrocyte separation.

5. Diagnostic test carrier as claimed in one of the previous claims, wherein the thickness of the first and second film layer in the dry state are together a maximum of 0.20 mm, preferably a maximum of 0.12 mm and particularly preferably a maximum of 0.08 mm.

6. Diagnostic test carrier as claimed in claim 5, wherein the second film layer is about 2-times to 5-times thicker than the first.

7. Diagnostic test carrier as claimed in one of the previous claims, wherein the colour forming reagent is located in the first film layer.

8. Diagnostic test carrier as claimed in one of the claims 1-6, wherein the components of the reagent system are distributed among both film layers.

9. Diagnostic test carrier as claimed in one of the previous claims, wherein the film layers do not contain a haemolyzing wetting agent.

10. Diagnostic test carrier as claimed in claim 9, wherein at least one film layer contains N-octanoyl-N-methyl-glucamide as the wetting agent.

11. Method for the determination of an analyte from whole blood with the aid of a diagnostic test carrier as claimed in one of the claims 1-10, wherein blood is applied to the sample application side of the test field and the detection side is observed for colour formation wherein the intensity of the colour formation is a measure of the amount of analyte in the blood.

12. Method as claimed in claim 11, wherein the detection side is observed continuously or at intervals for colour formation.

13. Method as claimed in claim 12, wherein the observation is carried out by means of an apparatus in the form of a series of measurements by measuring colour formation and determining the evaluation time as the point at which the sequential measurements of colour formation fall once below a predetermined absolute value or percentage difference.

14. Method as claimed in claim 12, wherein the observation is carried out by means of an apparatus in the form of a series of measurements by measuring colour formation and determining the time of evaluation as the point when successive measurements of colour formation fall several times below a predetermined absolute value or percentage difference.

15. Method as claimed in claim 13 or 14, wherein a further after-reaction period follows after falling once or several times below the absolute value of percentage difference.

16. Method as claimed in one of the claims 13 - 15, wherein the colour formation is measured reflectometrically and the last measured value of the measurement series is used to mathematically evaluate the analyte concentration.

## Revendications

1. Support d'essai diagnostique pour la détermination d'un analyte à partir de sang complet à l'aide d'un système réactif contenu dans le support, qui englobe un réactif de coloration, comprenant un champ d'essai qui présente un côté destiné à l'application de l'échantillon, sur lequel on amène l'échantillon de sang, et un côté de décèlement sur lequel a lieu une modification détectable par voie optique, suite à la réaction de l'analyte avec le système réactif, et qui est réalisé de telle sorte que les érythrocytes contenus dans l'échantillon n'aboutissent pas sur le côté destiné au décèlement, dans lequel le champ d'essai comprend une feuille transparente et une première couche pelliculaire et une deuxième couche pelliculaire appliquée sur la première couche pelliculaire, dans lequel la première couche disposée sur la feuille transparente possède, à l'état humide, un effet de diffusion de la lumière essentiellement inférieur à celui de la deuxième couche sus-jacente, et
dans lequel le côté non enduit de la feuille représente le côté destiné au décèlement et le côté de la deuxième couche qui est opposé au côté avec lequel la deuxième couche vient s'appliquer sur la première, représente le côté destiné à l'application de l'échantillon,
**caractérisé en ce que** la première couche pelliculaire contient une substance de charge dont l'indice de réfraction est proche de l'indice de réfraction de l'eau et **en ce que** la deuxième couche contient un pigment possédant un indice de réfraction d'au moins 2,5 en une concentration d'au moins 25 % en poids rapportés à la double couche pelliculaire à l'état séché.

2. Support d'essai diagnostique selon la revendication 1, **caractérisé en ce que** les deux couches pelliculaires sont formées à partir d'une dispersion ou d'une émulsion d'un polymère filmogène qui contient, à l'état distribué d'une manière homogène, le polymère filmogène et un agent de gonflement, l'aptitude au gonflement manifestée par ledit agent étant à ce point élevée que l'on peut mesurer la modification détectable par voie optique sur le côté destiné au décèlement après un temps maximal de une minute.

3. Support d'essai diagnostique selon l'une quelconque des revendications précédentes, **caractérisé en ce que** la première couche pelliculaire contient un silicate de sodium-aluminium à titre de substance de charge et la deuxième couche pelliculaire contient du dioxyde de titane à titre de pigment.

4. Support d'essai diagnostique selon l'une quelconque des revendications précédentes, **caractérisé en ce que** les deux couches pelliculaires sont appropriées pour la séparation d'érythrocytes.

5. Support d'essai diagnostique selon l'une quelconque des revendications précédentes, **caractérisé en ce que** l'épaisseur des première et deuxième couches pelliculaires, à l'état sec, s'élève de manière conjointe à une valeur maximale de 0,20 mm, de préférence à une valeur maximale de 0,12 mm, de manière particulièrement préférée à une valeur maximale de 0,08 mm.

6. Support d'essai diagnostique selon la revendication 5, **caractérisé en ce que** la deuxième couche pelliculaire possède une épaisseur d'environ 2 à 5 fois supérieure à celle de la première.

7. Support d'essai diagnostique selon l'une quelconque des revendications précédentes, **caractérisé en ce que** le réactif de coloration se trouve dans la première couche pelliculaire.

8. Support d'essai diagnostique selon l'une quelconque des revendications 1 à 6, **caractérisé en ce que** les composants du système réactif sont répartis entre les deux couches pelliculaires.

9. Support d'essai diagnostique selon l'une quelconque des revendications précédentes, **caractérisé en ce que** les couches pelliculaires sont exemptes d'agents mouillants ayant un effet hémolytique.

10. Support d'essai diagnostique selon la revendication 9, **caractérisé en ce qu'**au moins une couche pelliculaire contient, à titre d'agents mouillants, du N-octanoyl-N-méthyl-glucamide.

11. Procédé pour la détermination d'un analyte à partir de sang complet à l'aide d'un support d'essai diagnostique selon l'une quelconque des revendications 1 à 10, **caractérisé en ce qu'**on achemine du sang sur le côté du champ d'essai destiné à l'application de l'échantillon et on observe le côté destiné au décèlement pour détecter une coloration, l'intensité de la coloration représentant une mesure de la quantité de l'analyte dans le sang.

12. Procédé selon la revendication 11, **caractérisé en ce que** le côté destiné au décèlement est observé en continu ou par intervalles afin de détecter une coloration.

13. Procédé selon la revendication 12, **caractérisé en ce que** l'observation a lieu à l'aide d'un appareillage sous la forme d'une série de mesures via des mesures de la coloration, le moment de l'évaluation étant déterminé par le dépassement unique vers le bas d'une valeur déterminée de différence absolue ou selon un pourcentage de mesures successives de la coloration.

14. Procédé selon la revendication 12, **caractérisé en ce que** l'observation a lieu à l'aide d'un appareillage sous la forme d'une série de mesures via des mesures de la coloration, le moment de l'évaluation étant déterminé par le dépassement à plusieurs reprises vers le bas, fixé au préalable, d'une valeur déterminée de différence absolue ou selon un pourcentage de mesures successives de la coloration.

15. Procédé selon la revendication 13 ou 14, **caractérisé en ce que**, suite au dépassement unique ou à plusieurs reprises vers le bas de la valeur de différence absolue ou selon un pourcentage, un temps de réaction ultérieure supplémentaire suit.

16. Procédé selon l'une quelconque des revendications 13 à 15, **caractérisé en ce qu'**on procède aux mesures de la coloration à l'aide d'un réflectomètre et on utilise la dernière valeur de mesure de la série de mesures pour l'évaluation mathématique de la concentration de l'analyte.
